# FASCICULE DE BREVET EUROPEEN

(11) **EP 1 531 158 B1**
(45) Date de publication et mention de la délivrance du brevet: **13.09.2006**
(21) Numéro de dépôt: 04292619.6
(22) Date de dépôt: 04.11.2004
(51) Int. Cl.: C07H 13/06, A61K 8/60, A61Q 19/00, A61Q 19/08

(54) **Lipide A et composition topique, notamment cosmétique, le comprenant**
Lipid A und Lipid A enthaltende topische, insbesondere kosmetische, Zusammensetzung
Lipid A and topic, in particular cosmetic, composition thereof

(30) Priorité: 06.11.2003 FR 0350798
(43) Date de publication de la demande: 18.05.2005
(73) Titulaire: L'ORÉAL, 75008 Paris (FR)
(72) Inventeur: Martin, Richard, 37210 Rochecorbon (FR)
(74) Mandataire: Renard, Emmanuelle

(56) Documents cités:
- HASEGAWA N ET AL: "ELEVATED PROMOTION OF PROSTACYCLIN PRODUCTION BY SYNTHETIC LIPID A ANALOGS IN AGED HUMAN ENDOTHELIAL CELLS IN CULTURE" MECHANISMS OF AGEING AND DEVELOPMENT, ELSEVIER SEQUOIA, LAUSANNE,, CH, vol. 78, no. 2, 1995, pages 155-162, XP001181663 ISSN: 0047-6374
- TANAMOTO K ET AL: "ENDOTOXIC PROPERTIES OF LIPID A FROM COMAMONAS TESTOSTERONI" MICROBIOLOGY, SOCIETY FOR GENERAL MICROBIOLOGY, READING, GB, vol. 147, no. 5, 2001, pages 1087-1094, XP001181665 ISSN: 1350-0872
- BERARDESCA, E. ET AL: "Clinical and instrumental evaluation of the activity of an anti-wrinkle cosmetic product on cutaneous relief and photoaged skin" JOURNAL OF APPLIED COSMETOLOGY , 15(2), 69-75 CODEN: JACOEL; ISSN: 0392-8543, 1997, XP009033298

## Description

La présente invention se rapporte à un nouveau composé du type lipide A, ainsi qu'aux compositions le comprenant et à ses utilisations cosmétiques et dermatologiques.

La peau constitue l'organe le plus important de l'organisme et est reconnue comme l'un des principaux éléments actifs du système de défense immunitaire. Trois types de cellules épidermiques participent à ce système : les kératinocytes, les méianocytes et les cellules de Langerhans. Ces cellules, que l'on ne retrouve qu'au niveau de la peau, jouent un rôle primordial dans la réponse immunitaire et en particulier dans la présentation antigénique.

La peau saine est capable de se défendre des agressions extérieures grâce aux moyens mis à sa disposition. On sait toutefois que le système immunitaire, et plus particulièrement celui de la peau, s'affaiblit au cours du vieillissement chronobiologique.

En outre, la peau est soumise à l'agression permanente de l'environnement et de certains produits chimiques. En particulier, les cellules de Langerhans sont la cible privilégiée des rayonnements ultraviolets. Ces agressions se traduisent par un effet suppresseur des défenses immunitaires.

Celui-ci a notamment pour conséquence une moins bonne destruction des "sunburn cells" dont l'accumulation génère des cytokines qui sont susceptibles de générer elles-mêmes des radicaux libres et d'altérer le derme (en particulier en favorisant la dégradation du collagène) et l'épiderme (en particulier en ralentissant le renouvellement de l'épithélium). Ces effets concourent à une accélération du vieillissement cutané.

Un effet immunostimulateur peut alors rétablir les fonctions immunitaires et plus particulièrement celles de l'épiderme en renforçant les défenses naturelles de la peau et permettre ainsi notamment de lutter contre ou de prévenir les signes cutanés du vieillissement.

On connaît, dans l'art antérieur, des extraits bactériens dotés de propriétés immunostimulantes. Il est par ailleurs connu que les lipopolysaccharides (désignes ci-après par LPS) ancrés dans la membrane externe de ces bactéries sont pour partie responsables de ces propriétés.

Les LPS sont des molécules chimiques complexes, de masse moléculaire comprise entre 8.000 et 54.000 Daltons, ayant une structure étagée en trois compartiments qui, de l'extérieur vers l'intérieur de la cellule, sont :
- l'antigène O, qui est un polymère, spécifique à une souche donnée, constitué de 1 à 10 motifs dont chacun comprend un enchaînement de 5 à 7 sucres généralement aminés,
- le Core, qui est un polysaccharide très conservatoire d'un genre bactérien à l'autre et est relié au lipide A par une molécule spécifique des bactéries à Gram négatif, le KDO (acide 2-céto 3-déoxy octulosonique), et
- le lipide A qui ancre le LPS dans la membrane externe de la bactérie.

Le lipide A est un dimère de glucosamine porteur par condensation, sur ses groupes hydroxyles situés en positions 3 et 3', ainsi que sur ses groupes amino, situés en positions 2 et 2', d'acides gras plus ou moins insaturés et hydroxylés qui peuvent eux-mêmes êtres estérifiés, sur leurs groupes hydroxyles, par d'autres acides gras. Ce sont ces acides gras qui permettent l'ancrage du lipide A dans la membrane externe de la cellule, qui est de nature phospholipidique. En outre, leur nature (en C₁₂-C₁₈) et leur positionnement sur les glucosamines, déterminent et l'activité biologique des lipides A et donc des LPS.

La recherche de LPS biologiquement actifs se heurte toutefois au problème de l'endotoxicité de ces molécules. En effet, la plupart des LPS possèdent un lipide A qui, une fois désancré de la membrane cellulaire, est capable de se fixer aux récepteurs CD14 et de les activer, entraînant notamment ainsi un relarguage de TNF susceptible de provoquer un choc septique.

Des recherches ont donc été menées en vue de déterminer quels facteurs structuraux pouvaient influencer le caractère agoniste ou antagoniste des récepteurs CD14 de ces LPS. Il a ainsi été mis en évidence que les LPS de forme cylindrique et/ou dont les acides gras sont disposés symétriquement sur le squelette glucosamine sont plutôt des antagonistes de CD14, par opposition aux LPS de forme conique et/ou asymétriques.

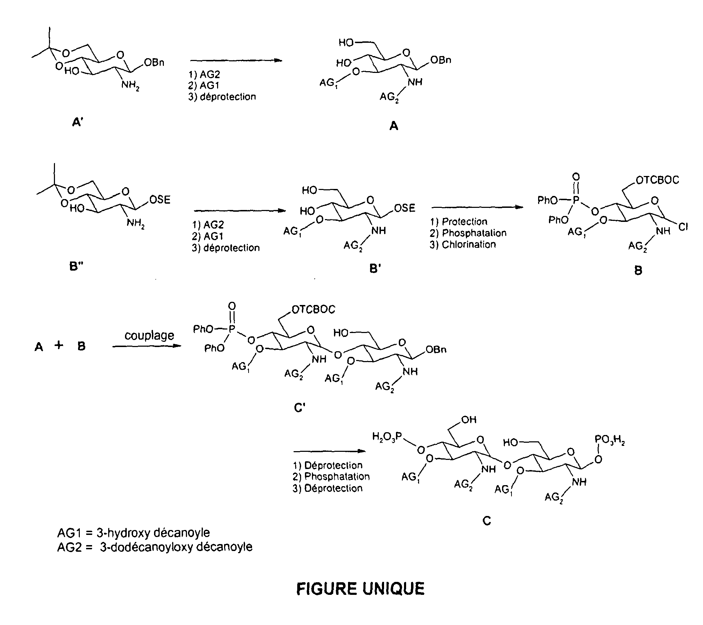

C'est dans ce contexte que la Demanderesse a découvert un nouveau lipide A doté de propriétés immunostimulantes tout en état dépourvu de toxicité. Ce lipide A a une structure très proche (à deux atomes de carbone près) de celui présent dans le LPS de *Neisseria meningitidis,* tel que décrit dans le brevet US-6,482,807 (Figure 4a), sans toutefois présenter sa toxicité.

Berardesca et al. (J. Appl. Cosmetol., 15, 69-75 (1997)) décrivent l'utilisation d'extraits de Vitreoscilla filiformes à des fins anti-rides et anti-âge, mans ne spécifie toutefois aucune formule.

Le lipide A selon l'invention a été initialement isolé à partir d'une souche de *Vitreoscilla filiformis.*

La souche bactérienne *Vitreoscilla filiformis* est utilisée actuellement par la Demanderesse pour la fabrication d'une biomasse introduite dans des produits cosmétiques. Le procédé de préparation de cette biomasse comprend la culture des bactéries en milieu stérile oxygéné, en présence de sels minéraux et de sucres ; le prélèvement, puis la centrifugation, du milieu de culture comprenant les bactéries, pour obtenir une biomasse qui est mise en flacons puis stérilisée. L'éclatement des cellules résultant de la stérilisation provoque une décantation de la biomasse en une boue renfermant essentiellement des membranes cellulaires et des protéines coagulées et contenant environ 70% du poids de LPS, et en un surnageant comprenant le cytoplasme et contenant environ 30% du poids de LPS, la bactérie entière contenant (en poids sec) environ 10% de LPS. Une biomasse homogène est reconstituée par agitation avant emploi.

Divers extraits de *Vitreoscilla filiformis,* dotés de propriétés immunostimulantes et non toxiques, ont été décrits par la Demanderesse en vue d'une utilisation cosmétique ou pharmaceutique, en particulier dans le traitement des signes cutanés du vieillissement.

Ainsi, la demande EP-0 765 667 utilise une fraction de bactéries riche en ribosomes, obtenue par centrifugation de la biomasse et dialyse du surnageant obtenu. La demande EP-0 876 813 décrit en outre une fraction immunostimulante, obtenue à partir du milieu de culture de ces bactéries. Enfin, la demande WO 94/02158 divulgue l'utilisation comme stimulant immunitaire d'enveloppes de bactéries ou de fractions, notamment du LPS, obtenues à partir desdites enveloppes.

Toutefois, les fractions décrites dans l'art antérieur ci-dessus ne renfermaient qu'une très faible quantité, indétectable en pratique, de lipide A libre, dont la structure n'avait jamais été élucidée à ce jour, l'essentiel du lipide A se trouvant sous forme de LPS lui-même présent en relativement faible quantité dans la plupart des extraits de l'art antérieur.

En outre, il n'était pas évident que les effets immunostimulants des fractions bactériennes de l'art antérieur étaient liées à la présence de LPS. En effet, le LPS n'est présent qu'en faible quantité à côté d'autres immunogènes que sont notamment la muréine contenue dans les membranes cellulaires ou les ribosomes présents dans le cytoplasme (surnageant). En outre, on pouvait penser que le traitement thermique utilisé pour récupérer ces fractions (121°C pendant 40 mn) avait dénaturé le LPS, le rendant ainsi inactif. Surtout, il n'était pas prévisible que les effets immunostimulants des fractions bactériennes de l'art antérieur pouvaient être conservées par le LPS qu'elles contenaient, sans risque de toxicité, après que celui-ci ait été séparé des membranes cellulaires donc rendu potentiellement actif vis-à-vis des récepteurs CD14.

C'est donc de façon surprenante que la Demanderesse, ayant identifié le lipide A présent dans le LPS de *Vitreoscilla filiformis,* a montré qu'il présentait une activité immunostimulante tout en étant dépourvu de toxicité. Cette découverte a permis à la Demanderesse d'envisager son utilisation dans le domaine cosmétique où, contrairement au domaine pharmaceutique, on recherche des composés actifs présentant une parfaite tolérance.

L'invention a par conséquent pour objet un nouveau lipide A caractérisé en ce qu'il est
constitué d'un composé choisi parmi : (a) les composés de formule (I) : 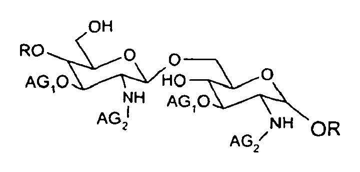 dans laquelle :
   AG₁ désigne un groupe 3-hydroxy décanoyle
   AG₂ désigne un groupe 3-dodécanoyloxy décanoyle, où R désigne un atome d'hydrogène ou un groupe PO(OR')₂, R' désignant un atome d'hydrogène, un groupe alkyle linéaire ou ramifié, saturé ou insaturé, en C₁-C₆, ou un groupe phényle ou benzyle, et
   (b) dans le cas où R désigne un groupe PO(OH)₂ dans la formule (I) ci-dessus, d'un sel inorganique du composé de formule (i), ou d'un sel d'aminé primaire, secondaire ou tertiaire du composé de formule (I), ou d'un sel de phosphoéthanolamine du composé de formule (I),
étant entendu que, lorsque plusieurs groupements R, respectivement R', sont présents, ils sont identiques ou différents les uns des autres.

Le sel d'aminedu composé de formule (I) peut être choisi parmi les sels de mono-, di- et triéthanolamine, les sels de mono-, di- ou tri-isopropanol-amine, le 2-amino 2-méthyl 1-propanol, le 2-amino 2-méthyl 1,3-propanediol et le tris(hydroxyméthyl)amino méthane.

Les sels inorganiques du composé de formule (I) peuvent notamment être des sels de sodium, magnésium, potassium, zinc ou calcium.

L'invention a également pour objet une composition adaptée à une application topique sur la peau, renfermant, dans un milieu cosmétiquement acceptable, un tel lipide A.

Par cosmétiquement acceptable, on entend au sens de la présente invention un milieu inerte qui n'entraîne pas de démangeaisons, de picotements ou de rougeurs susceptibles de détourner l'utilisateur de la composition, et qui présente un aspect, une odeur et un toucher agréables.

La présente invention a encore pour objet l'utilisation cosmétique d'un lipide A tel que défini précédemment, ou de la composition précitée, pour le soin de la peau, en particulier pour prévenir ou traiter les signes cutanés du vieillissement et/ou pour protéger la peau contre les méfaits des UV.

Elle a également pour objet un procédé de traitement cosmétique de la peau, comprenant l'application topique sur la peau de la composition précitée. Ce procédé est en particulier destiné à prévenir ou traiter les signes cutanés du vieillissement et/ou à protéger la peau contre les méfaits des UV.

Elle a encore pour objet l'utilisation du lipide A tel que défini précédemment pour la préparation d'une composition dermatologique destinée à renforcer les défenses immunitaires cutanées.

Le lipide A selon l'invention peut être préparé par synthèse chimique, selon le procédé illustré sur la Figure annexée et décrit plus en détail à l'Exemple 1 ci-après.

En variante, il peut être obtenu à partir d'une culture de bactéries filamenteuses non photosynthétiques, non fructifiantes.

Dans cette variante du procédé, on peut utiliser une culture de bactéries de l'ordre des Beggiatoales, par exemple du genre Beggiatoa, telles que diverses souches de Beggiatoa alba selon la définition donnée dans Arch. Microbiol. (1984) 137, 139-144. On peut également utiliser une culture de bactéries du genre Vitreoscilla, Flexithrix ou Leucothrix. Ces bactéries peuvent se trouver dans l'eau ou mer ou dans certaines sources thermales.

Parmi les bactéries utilisables, on peut notamment citer :
- *Vitreoscilla beggiatoides* (ATCC 43181)
- *Vitreoscilla stercoraria* (ATCC 15218)
- *Vitreoscilla beggiatoides* (ATCC 43181)
- *Beggiatoa alba* (ATCC 33555)
- *Flexithrix dorothaea* (ATCC 23163)
- *Leucothrix mucor* (ATCC 25107).

Ces bactéries peuvent être cultivées selon des procédés connus, tels que décrits par exemple dans la demande WO 94/02158, pour obtenir une biomasse qui peut être séparée et isolée de différentes manières, par exemple par filtration ou centrifugation. On extrait ensuite de cette biomasse une fraction lipopolysaccharidique, par exemple selon la méthode dite de Westphal (Westphal, O. and Jann, K. (1965), in R.L. Whistler (ed.) Methods in Carbohydrate Chemistry Vol. 5, Academic Press, New York, pp. 83-91). Cette méthode comprend une extraction à l'aide de mélanges phénol-eau à 65°C suivie d'une dialyse en vue d'éliminer le phénol. On obtient ainsi une fraction bactérienne concentrée en LPS. Le lipide A selon l'invention peut ensuite être obtenu à partir à partir de cette fraction, par exemple suivant l'une des méthodes décrites dans Morrison, D. C. et Leive, L. (1975) J. Biol. Chem. 250, 2911-2919 ou Takayama, K. et (1981) Cancer Research 41, 2654-2657.

La composition selon la présente invention renferme une quantité suffisante de lipide A pour obtenir l'effet recherché et de préférence de 0,01 à 0,1% en poids de ce composé, par rapport au poids total de la composition.

La composition selon l'invention peut se présenter sous toutes les formes galéniques classiquement utilisées pour une application topique et notamment sous forme de gels aqueux, de solutions aqueuses ou hydroalcooliques. Elles peut aussi, par ajout d'une phase grasse ou huileuse, se présenter sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un mode préféré de réalisation de l'invention, la composition se présente sous forme d'une émulsion.

La composition utilisée selon l'invention peut être plus ou moins fluide et avoir l'aspect d'une crème blanche ou colorée, d'une pommade, d'un lait, d'une lotion, d'un sérum, d'une pâte, d'une mousse. Elle peut être éventuellement appliquée sur le contour de l'oeil sous forme solide, et par exemple sous forme coulée, en coupelle ou sous forme de stick.

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; et leurs mélanges.

La composition selon l'invention peut également contenir les adjuvants habituels dans les domaines cosmétique et dermatologique, tels que les gélifiants hydrophiles ou lipophiles, les actifs, les filtres, les conservateurs, les solvants, les parfums, les charges, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses de selon l'invention.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, la composition selon l'invention peut notamment comprendre au moins un composé choisi parmi : les agents desquamants ou hydratants ; les agents dépigmentants ; ies agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes ou des kératinocytes et/ou la différenciation des kératinocytes ; les agents tenseurs ; les agents anti-pollution et/ou anti-radicalaires ; et les agents myorelaxants ou dermo-décontractants.

Des exemples d'actifs préférés pour une utilisation dans la présente invention sont : l'acide (N-2 hydroxyéthylpiperazine-N-2-éthane) sulfonique (HEPES) ; l'acide 2-oxothiazolidine-4-carboxylique (procystéine) ; les α- et β-hydroxyacides, en particulier l'acide n-octanoyl-5-salicylique ; les céramides ; les sapogénines et les extraits végétaux, en particulier de Wild Yam, en contenant ; le resvératrol ; l'acide ascorbique et ses dérivés ; les rétinoïdes et caroténoïdes, en particulier le rétinol, les esters de rétinyle et le lycopène ; les pseudodipeptides tels que l'acide {2-[acétyl-(3-trifluorométhyl-phényl)-amino]-3-méthyl-butyrylamino} acétique ; les extraits de soja, en particulier les hydrolysats de protéine de soja ou les extraits de soja riches en isoflavones ; le tocophérol et ses esters ; et leurs mélanges.

Comme filtres utilisables dans la présente invention, on peut citer les agents photoprotecteurs organiques ou inorganiques, actifs dans l'UVA et/ou l'UVB, liposolubles, hydrosolubles ou insolubles dans les solvants cosmétiques.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; ies 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs actifs dans l'UV-A et/ou l'UV-B, on peut citer désignés ci-dessus sous leur nom INCI :
- les dérivés de l'acide para-aminobenzoïique, dont les suivants : PABA, Ethyl PABA, Ethyl Dihydroxypropyl PABA, Ethylhexyl Diméthyl PABA vendu notamment sous le nom « ESCALOL 507 » par ISP, Glyceryl PABA, PEG-25 PABA vendu sous le nom « UVINUL P25 » par BASF,
- les dérivés salicyliques, dont les suivants : Homosalate vendu notamment sous le notamment sous le nom « NEO HELIOPAN OS » par HAARMANN et REIMER, Dipropyleneglycol Salicylate vendu notamment sous le nom « DIPSAL » par SCHER, TEA Salicylate vendu notamment sous le nom « NEO HELIOPAN TS » par HAARMANN et REIMER,
- les dérivés du dibenzoylméthane, dont les suivants: Butyl Methoxydibenzoylmethane vendu notamment sous le nom commercial « PARSOL 1789 » par HOFFMANN LAROCHE, Isopropyl Dibenzoylmethane,
- les dérivés cinnamiques, dont les suivants :Ethylhexyl Methoxycinnamate vendu notamment sous le nom commercial « PARSOL MCX » par HOFFMANN LAROCHE, Isopropyl Methoxy cinnamate, Isoamyl Methoxy cinnamate vendu notamment sous le nom commercial « NEO HELIOPAN E 1000 » par HAARMANN et REIMER, Cinoxate, DEA Methoxycinnamate, Diisopropyl Methylcinnamate, Glyceryl Ethylhexanoate Dimethoxycinnamate,
- les dérivés de β,β'-diphénylacrylate, dont les suivants : Octocrylene vendu notamment sous le nom commercial « UVINUL N539 » par BASF, Etocrylene vendu notamment sous le nom commercial « UVINUL N35 » par BASF,
- les dérivés de la benzophénone, dont les suivants : Benzophenone-1 vendue notamment sous le nom commercial « UVINUL 400 » par BASF, Benzophenone-2 vendue notamment sous le nom commercial « UVINUL D50 » par BASF, Benzophenone-3 ou Oxybenzone vendue notamment sous le nom commercial « UVINUL M40 » par BASF, Benzophenone-4 vendue notamment sous le nom commercial « UVINUL MS40 » par BASF, Benzophenone-5, Benzophenone-6 vendue notamment sous le nom commercial « HELISORB 11 » par NORQUAY, Benzophenone-8 vendue notamment sous le nom commercial «SPECTRA-SORB UV-24 » PAR AMERICAN CYANAMID, Benzophenone-9 vendue notamment sous le nom commercial« UVINUL DS-49» par BASF, Benzophenone-12, et le 2-(4-diéthylamino-2-hydroxybenzoyl)-benzoate de n-hexyle
- les dérivés du benzylidène camphre, dont les suivants : 3-Benzylidene camphor, 4-Methylbenzylidene camphor vendu notamment sous le nom « EUSOLEX 6300 » par MERCK, Benzylidene Camphor Sulfonic Acid, Camphor Benzalkonium Methosulfate, Terephthalylidene Dicamphor Sulfonic Acid, Polyacrylamidomethyl Benzylidene Camphor,
- les dérivés de benzimidazole, dont les suivants : Phenylbenzimidazole Sulfonic Acid vendu notamment sous le nom commercial « EUSOLEX 232 » par MERCK, Disodium Phenyl Dibenzimidazole Tetra-sulfonate vendu notamment sous le nom commercial commercial « NEO HELIOPAN AP » par HAARMANN et REIMER,
- les dérivés de triazine, dont les suivants : Anisotriazine vendu notamment sous le nom commercial «TINOSORB S » par CIBA SPECIALTY CHEMICALS, Ethylhexyl triazone vendu notamment sous le nom commercial «UVINUL T150 » par BASF, Diethylhexyl Butamido Triazone vendnotamment sous le nom commercial « UVASORB HEB » par SIGMA 3V, et la 2,4,6- tris-(4'aminobenzalmalonate de diisobutyle)-s-triazine,
- les dérivés de benzotriazole, dont les suivants : Drometrizole Trisiloxane vendu sous le nom « SILATRIZOLE » par RHODIA CHIMIE, Methylène bis-Benzotriazolyl Tetramethylbutylphénol, vendu notamment sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,
- les dérivés anthranilique, dont le Menthyl anthranilate vendu sous le nom commercial commercial « NEO HELIOPAN MA » par HAARMANN et REIMER,
- les dérivés d'imidazolines, dont l'Ethylhexyl Dimethoxybenzylidene Dioxoimidazoline Propionate.

On peut citer également les dérivés de benzalmalonate, dont le polyorganosiloxane à fonctions benzalmalonate vendu sous la dénomination commerciale « PARSOL SLX » par HOFFMANN LAROCHE, ainsi que les dérivés de 4,4-diaryibutadiène, dont le 1,1-dicarboxy (2,2'-diméthyl-propyl)-4,4-diphénylbutadiène.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres sont généralement présents dans les compositions selon l'invention dans des proportions allant de 0,1 à 20% en poids par rapport au poids total de la composition, et de préférence allant de 0,2 à 15% en poids par rapport au poids total de la composition.

L'invention sera maintenant illustrée par les exemples non limitatifs suivants. Dans ces exemples, les quantités sont indiquées en pourcentage pondéral. Les constituants des compositions sont désignés selon la nomenclature CTFA.

### EXEMPLES

### Exemple 1 : Préparation du lipide A

### a) Synthèse de A

Une solution contenant le glucosamine commercial **A'** (1.4 mmol) et le 3-dodécanoyloxy décanoyle (1.54 mmol) dans CH₂Cl₂ (15 ml) est traitée avec EDC.Mel (2.10 mmol) et agitée à température ambiante toute la nuit. Le mélange réactionnel est ensuite concentré et le résidu est purifié par chromatographie flash sur gel de silice.

Le composé précédent (15,2 mmol) est mis en solution avec le 3-hydroxy décanoyle (16.7 mmol) et la 4-pyrrolidinopyridine (1.7 mmol) dans CH₂Cl₂ (95 mL). EDC.Mel (16.7 mmol) est ajouté et le milieu est agité à température ambiante toute la nuit. Le mélange réactionnel est ensuite concentré et le résidu est purifié par chromatographie flash sur gel de silice.

L'acétonide obtenu ci-dessus est dissout dans un mélange acide acétique / eau (4 / 1) et chauffé à 60°C pendant 1 h. Le milieu est ensuite concentré et purifié par chromatographie flash sur gel de silice pour obtenir l'intermédiaire **A**.

### b) Synthèse de B

**B**' est obtenu à partir du produit commercial **B**" en appliquant le même protocole utilisé pour la synthèse de **A** (voir ci-dessus).

TCBOC-Cl (13.2 mmol) dans CH₂Cl₂ (25 mL) est ajouté goutte à goutte pendant 15 min, à une solution à 0°C contenant **B'** (12 mmol) et pyridine (25 mmol) dans CH₂Cl₂ (125 ml). Le mélange est ensuite ramené doucement à température ambiante pendant 3,5 h. On ajoute successivement la 4-pyrrolidinopyridine (6.0 mmol), la N,N-diisopropyléthylamine (60 mmol), et le diphényl chlorophosphonate (18 mmol). Le mélange est agité à température ambiante pendant 5h. La réaction est alors diluée avec CH₂Cl₂, lavée avec une solution aqueuse de HCl (7.5 %) froide, puis avec une solution aqueuse saturée en NaHCO₃, puis séchée et concentrée. Le résidu est purifié par chromatographie flash sur gel de silice.

ZnCl₂ (1.0 M dans éther, 2.41 mmoi) est ajouté à 0 °C à une solution contenant !e composé obtenu ci-dessus (4.84 mmol) et du dichlorométhyl méthyl éther (24.2 mmol) dans CHCl₃ (60 mL). Le mélange est amené doucement à température ambiante, puis agité à température ambiante toute la nuit. Le milieu réactionnel est ensuite dilué avec EtOAc, lavé avec une solution aqueuse saturée en NaHCO₃, puis séchée et concentrée. Le résidu est purifié par chromatographie flash sur gel de silice pour obtenir le composé **B**.

### c) Synthèse de C

Une solution contenant **B** (1.85 mmol) et **A** (1.54 mmol) dansle 1,2-dichloroéthane (18.5 mmol) est agitée avec du tamis moléculaire 4 A (1g) pendant 1h, puis traitée avec AgOTf (5.55 mmol) en une seule portion. Après avoir agité 4h à température ambiante dans le noir, un supplément d'AgOTf (1.85 mmol) est ajouté et la réaction est agitée toute la nuit. Le mélange crémeux est ensuite filtré sur célite et concentré. Une purification par chromatographie flash sur gel de silice permet d'obtenir le composé **C**'.

Une solution contenant **C**' (0.46 mmol) dans un mélange AcOH (4.5 mL) / THF (40 mL) est hydrogénée en présence de PtO₂ (0.45 g) à température ambiante sous une pression de 70 psig pendant 18 h. La solution est diluée avec CHCl₃/MeOH (2/1), puis soniquée brièvement. Le catalyseur est ensuite filtré et lavé avec le mélange CHCl₃/MeOH (2/1). Les filtrats sont regroupés et concentrés. Le résidu est purifié par chromatographie flash sur gel de silice.

Le produit précédent (12 mmol) dans CH₂Cl₂ (25 mL) est agité en présence de N,N-diisopropyléthylamine (60 mmol), et POCl₃ (18 mmol) à température ambiante pendant 5h. La réaction est ensuite concentrée. Le milieu réactionnel est ensuite dilué avec CH₂Cl₂, lavé avec une solution aqueuse saturée en NaHCO₃, puis séché et concentré. Le résidu est ensuite dissout dans l'acide acétique (100 mL) et chauffé à 60°C avec de la poussière de Zinc (0.9 mmol). La réaction est ensuite refroidie et filtrée sur célite, puis concentrée. Une purification par chromatographie flash sur gel de silice permet d'obtenir le composé **C**.

### Exemple 2 : Mise en évidence de l'effet immuno-stimulant

L'effet immunostimulant a été évalué sur une fraction de *Vitreoscilla filiformis* riche en LPS dont le lipide A est conforme à la présente invention.

### a) Extraction du LPS

Une souche de *Vitreoscilla filiformis* (ATCC 15551) est mise en culture selon le procédé décrit dans la demande de brevet WO-9402158. La culture s'effectue à 26°C durant au moins 48 heures jusqu'à l'obtention d'une concentration cellulaire convenable correspondant à une densité optique à 600 nm supérieure ou égale à 1,5. On repique la souche à 2 % VN dans du milieu neuf toutes les 48 heures jusqu'à l'obtention d'une culture stable. Un Erlenmeyer de 1 litre contenant 200 ml de milieu neuf est alors ensemencé avec 4 ml de la culture précédente.

La culture en Erlenmeyer s'effectue à 26° C sur une table de culture agitée à 100 tours/minutes. Le pied de cuve ainsi obtenu sert d'inoculum à un fermenteur de 10 I. La croissance s'effectue à 26° C, pH 7, 100 tours/minute et pO₂ ≥ 15 %.

Après 48 heures de croissance, la biomasse est transférée dans un fermenteur de 600 litres utiles, pour être cultivée dans les mêmes conditions.

On a utilisé le milieu de culture suivant :

| COMPOSITION | CONCENTRATION |
|---|---|
| Extrait autolytique de levure Biokar (réf. 112002) | 2,0 g/l |
| Peptone papaïnique de soja (origine PPS-USP Biokar réf. 1 1601) | 2,0 g/l |
| Micro-éléments de Heller | 2,0 ml/l |
| Glucose anhydre | 2,0 g/l |
| CaCl₂, 10 H₂O | 0,066 g/l |
| Eau distillée | 100,0 ml |

Le pH est ajusté à 7,15 par addition de soude ou potasse 1N avant stérilisation à 121°C pendant 20 min.

La composition des micro-éléments de Heller, pour 1I d'eau distillée, est la suivante :

| | |
|---|---|
| ZnSO₄, 7 H₂O | 1g |
| MnSO₄,H₂O | 0,076 g |
| CuSO₄, 5H₂O | 0,003 g |
| Kl | 0,010 g |
| H₃BO₃ | 1 g |
| AlCl₃,6H₂O | 0,050 g |
| NiCl₂,6H₂O | 0,030 g |

On ajoute à ce milieu de culture 0,2 g/l d'un antimousse de type polyméthylsiloxane (Silbione 97350 RP). La température est régulée entre 26 et 30°C, l'optimum se situant à 29°C.

Un cycle complet de croissance s'effectue en 48h environ. L'aération est régulée par un débitmètre massique pour avoir au minimum 20 % d'oxygène dissous. Il n'y a pratiquement plus de glucose résiduel en fin de croissance.

La séparation de la biomasse est effectuée par centrifugation. On opère dans une centrifugeuse de type industriel refroidie à 4°C permettant d'obtenir un pouvoir séparatif équivalent à 8000 g, imprimé pendant 2 minutes.

Le milieu de culture ainsi récolté est ensuite congelé pour une utilisation différée.

Avant utilisation, le milieu est décongelé à 4°C, puis les cellules sont transférées dans des emballagaes de 1 à 2 litres fermés qui sont autoclavés à 121 °C pendant une période de temps comrpise entre 20 et 40 minutes.

420 ml de produit autoclavé sont ensuite centrifugés à 100.000 g 4°C pendant 1 h 30. Les culots sont récupérés puis resuspendus dans 150 ml de tampon de solubilisation. Après chauffage à 100°C pendant une heure, on effectue une centrifugation à 10.000 g 4°C pendant 30 minutes. On prélève 100 ml de du surnageant auquel on ajoute 60 mg de protéinase K (0,6 mg/ml). Après chauffage à 60°C pendant une heure, on ajoute 2ûû mi d'une solution de chlorure de magnésium dans l'éthanol et on maintient la composition obtenue à -20°C pendant une nuit. On effectue ensuite deux précipitations à l'éthanol à -20°C. Après re-suspension dans l'eau distillée, le produit obtenu est dialysé contre l'eau puis lyophilisé.

### b) Expression des molécules de surface des cellules de Langerhans

Des cellules de Langerhans humaines normales, obtenues par traitement enzymatique, sont incubées pendant 18 heures avec 100 µg/ml du produit obtenu à l'étape (a) ci-dessus ou 100 µg/ml de LPS d'E. Coli, utilisé comme contrôle positif.

L'expérience est répétée trois fois.

L'expression de la molécule HLA-DR est exprimée en terme forme de rapport d'intensité moyenne de fluorescence. La moyenne de celui-ci, sur les trois expériences, est de 1,28 pour le produit testé, non significativement différente de celle mesurée pour le LPS de référence (1,19).

En outre, l'expression des molécules B7-2, appréciée par microscopie électronique et exprimée par le nombre de grains d'or pour 100 µm de membrane, est bien plus forte lorsque les cellules sont incubées avec le produit testé (162,4 ± 13,7) qu'avec le LPS de référence (88,5 ± 8,3).

Enfin, la capacité de phagocytose de billes de latex par les cellules de Langerhans, exprimée en nombre de billes phagocytées pour 100 µm² de membrane, est significativement augmentée en présence du produit testé (54,1 ± 11,4) et supérieure a ce qui est obtenu en présence du LPS de référence (34,7 ± 6,9).

Conclusion : le produit testé induit à la surface des cellules de Langerhans une augmentation d'expression des principales molécules impliquées dans le contact avec les lymphocytes T, nécessaire à l'initiation de la réponse immune. En outre, la phagocytose étant l'une des premières étapes de l'activité fonctionnelle des cellules de Langerhans à assurer la défense de l'organisme vis-à-vis d'agents pathogènes bactériens ou viraux, sa stimulation par le LPS testé laisse supposer une activité accrue de l'élimination d'agents pathogènes par les cellules de Langerhans, attribuable au lipide A de ce LPS.

### Exemple 3 : Composition cosmétique

On prépare une émulsion H/E à partir des ingrédients suivants, dans les proportions pondérales indiquée ci-dessous :

| | | |
|---|---|---|
| Stéarate de glycéryle | | 2,0 % |
| Polysorbate 60 | | 2,0 % |
| Acide stéarique | | 1,5 % |
| Hydroxyde de sodium | | 0,7 % |
| Carbomer | | 0,4 % |
| Huile de tournesol | | 15,0 % |
| Propylène glycol | | 3,0 % |
| Conservateurs | | 0,5 % |
| Lipide A* | | 0,1 % |
| Eau | qsp | 100 % |

| | | |
|---|---|---|
| *obtenu comme décrit à l'Exemple 1 | | |

Cette composition peut être préparée de manière classique pour l'homme du métier. Son procédé de préparation comprend de préférence une étape de dissolution du lipide A dans une solution aqueuse d'hydroxyde de sodium.

## Revendications

1. Lipide A **caractérisé en ce qu'**il est constitué d'un composé choisi parmi :
(a) les composés de formule (I) : 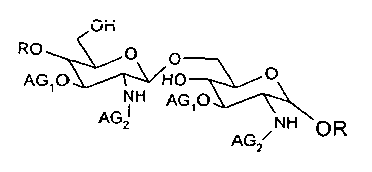 dans laquelle :
AG₁ désigne un groupe 3-hydroxy décanoyle
AG₂ désigne un groupe 3-dodécanoyloxy décanoyle,
où R désigne un atome d'hydrogène ou un groupe PO(OR')₂, R' désignant un atome d'hydrogène, un groupe alkyle linéaire ou ramifié, saturé ou insaturé, en C₁-C₆, ou un groupe phényle ou benzyle, et
(b) dans le cas où R désigne un groupe PO(OH)₂ dans la formule (I) ci-dessus, d'un sel inorganique du composé de formule (I), ou d'un sel d'amine primaire, secondaire ou tertiaire du composé de formule (I), ou d'un sel de phosphoéthanolamine du composé de formule (I),
étant entendu que, lorsque plusieurs groupements R, respectivement R', sont présents, ils sont identiques ou différents les uns des autres.

2. Lipide A selon la revendication 1, **caractérisé en ce que** le sel d'amine est choisi parmi les sels de mono-, di- et triéthanolamine, les sels de mono-, di- ou tri-isopropanol-amine, le 2-amino 2-méthyl 1-propanol, le 2-amino 2-méthyl 1,3-propanediol et le tris(hydroxyméthyl)amino méthane.

3. Lipide A selon la revendication 1, **caractérisé en ce que** le sel inorganique est un sel de sodium, magnésium, potassium, zinc ou calcium.

4. Composition adaptée à une application topique sur la peau, renfermant, dans un milieu cosmétiquement acceptable, un lipide A selon l'une quelconque des revendications 1 à 3.

5. Composition selon la revendication 4, **caractérisée en ce qu'**elle renferme de 0,01 à 0,1% en poids de lipide A selon la revendication 1, par rapport au poids total de la composition.

6. Composition selon la revendication 4 ou 5, **caractérisée en ce qu'**elle renferme en outre au moins un filtre UV organique ou inorganique.

7. Utilisation cosmétique d'un lipide A, tel que défini dans la revendication 1, ou d'une composition selon l'une quelconque des revendications 4 à 6, pour prévenir ou traiter les signes cutanés du vieillissement et/ou pour protéger la peau contre les méfaits des UV.

8. Procédé de traitement cosmétique de la peau, comprenant l'application topique sur la peau de la composition selon l'une quelconque des revendications 4 à 6.

9. Procédé selon la revendication 8, **caractérisé en ce qu'**il est destiné à prévenir ou traiter les signes cutanés du vieillissement et/ou à protéger la peau contre les méfaits des UV.

10. Utilisation du lipide A selon l'une quelconque des revendications 1 à 3 pour la préparation d'une composition dermatologique destinée à renforcer les défenses immunitaires cutanées.

## Claims

1. Lipid A, **characterized in that** it consists of a compound chosen from:
(a) the compounds of formula (I): 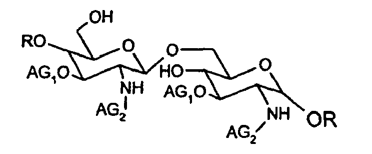 in which:
AG₁ denotes a 3-hydroxydecanoyl group
AG₂ denotes a 3-dodecanoyloxydecanoyl group,
where R denotes a hydrogen atom or a group PO(OR')₂, R' denoting a hydrogen atom, a saturated or unsaturated, linear or branched C₁-C₆ alkyl group, or a phenyl or benzyl group, and
(b) in the case where R denotes a group PO(OH)₂ in formula (I) above, an inorganic salt of the compound of formula (I), or a primary, secondary or tertiary amine salt of the compound of formula (I), or a phosphoethanolamine salt of the compound of formula (I),
it being understood that, when several groups R, respectively R', are present, they are identical or different from each other.

2. Lipid A according to Claim 1, **characterized in that** the amine salt is chosen from the mono-, di- and triethanolamine salts, the mono-, di- or triisopropanolamine salts, 2-amino-2-methyl-1-propanol, 2-amino-2-methyl-1,3-propanediol and tris(hydroxy-methyl)aminomethane.

3. Lipid A according to Claim 1, **characterized in that** the inorganic salt is a sodium, magnesium, potassium, zinc or calcium salt.

4. Composition, suitable for topical application to the skin, containing, in a cosmetically acceptable medium, a lipid A according to any one of Claims 1 to 3.

5. Composition according to Claim 4, **characterized in that** it contains from 0.01 to 0.1% by weight of lipid A according to Claim 1, relative to the total weight of the composition.

6. Composition according to Claim 4 or 5, **characterized in that** it additionally contains at least one organic or inorganic UV-screening agent.

7. Cosmetic use of a lipid A, as defined in Claim 1, or of a composition according to any one of Claims 4 to 6, to prevent or treat the cutaneous signs of ageing and/or to protect the skin against the damaging effects of UV radiation.

8. Method for the cosmetic treatment of the skin, comprising the topical application, to the skin, of the composition according to any one of Claims 4 to 6.

9. Method according to Claim 8, **characterized in that** it is intended to prevent or treat the cutaneous signs of ageing and/or to protect the skin against the damaging effects of UV radiation.

10. Use of the lipid A according to any one of Claims 1 to 3 for the preparation of a dermatological composition intended to strengthen the cutaneous immune defences.

## Patentansprüche

1. Lipid A, **dadurch gekennzeichnet, dass** es aus einer Verbindung besteht, die ausgewählt ist unter:
(a) den Verbindungen der Formel (I): 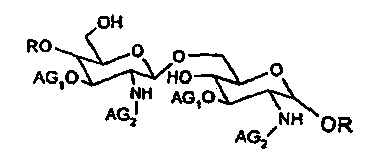 in der bedeuten:
AG₁ eine 3-Hydroxydecanoylgruppe
AG₂ eine 3-Dodecanoyloxydecanoylgruppe,
wobei R ein Wasserstoffatom oder eine Gruppe PO(OR')₂ bedeutet, worin R' ein Wasserstoffatom, eine geradkettige oder verzweigte, gesättigte oder ungesättigte C₁₋₆-Alkylgruppe oder eine Phenyl- oder Benzylgruppe ist, und
(b) wenn in der oben angegebenen Formel (I) R eine Gruppe PO(OH)₂ bedeutet, einem anorganischen Salz der Verbindung der Formel (I), einem primären, sekundären oder tertiären Aminsalzes der Verbindung der Formel (I) oder einem Phosphoethanolaminsalzes der Verbindung der Formel (I),
mit der Maßgabe, dass, wenn mehrere Gruppen R bzw. R' vorliegen, diese identisch oder voneinander verschieden sind.

2. Lipid A nach Anspruch 1, **dadurch gekennzeichnet, dass** das Aminsalz unter den Mono-, Di- und Triethanolaminsalzen, Mono-, Di- oder Triisopropanolaminsalzen, 2-Amino-2-methyl-1-propanol, 2-Amino-2-methyl-1,3-propandiol und Tris(hydroxymethyl)aminomethan ausgewählt ist.

3. Lipid A nach Anspruch 1, **dadurch gekennzeichnet, dass** das anorganische Salz ein Natriumsalz, Magnesiumsalz, Kaliumsalz, Zinksalz oder Calciumsalz ist.

4. Zusammensetzung, die für eine topische Anwendung auf die Haut geeignet ist und in einem kosmetisch akzeptablen Medium ein Lipid A nach einem der Ansprüche 1 bis 3 enthält.

5. Zusammensetzung nach Anspruch 4, **dadurch gekennzeichnet, dass** sie 0,01 bis 0,1 Gew.-% Lipid A nach Anspruch 1, bezogen auf das Gesamtgewicht der Zusammensetzung, enthält.

6. Zusammensetzung nach Anspruch 4 oder 5, **dadurch gekennzeichnet, dass** sie ferner mindestens ein organisches oder anorganisches UV-Filter enthält.

7. Kosmetische Verwendung eines Lipids A nach Anspruch 1 oder einer Zusammensetzung nach einem der Ansprüche 4 bis 6, um die Anzeichen der Hautalterung zu behandeln oder ihnen vorzubeugen und/oder die Haut gegen die schädlichen Wirkungen der UV-Strahlung zu schützen.

8. Verfahren zur kosmetischen Behandlung der Haut, das das topische Aufbringen der Zusammensetzung nach einem der Ansprüche 4 bis 6 auf die Haut umfasst.

9. Verfahren nach Anspruch 8, **dadurch gekennzeichnet, dass** es dazu dienen soll, den Anzeichen der Hautalterung vorzubeugen oder sie zu behandeln und/ oder die Haut gegenüber den schädlichen Wirkungen der UV-Strahlung zu schützen.

10. Verwendung des Lipids A nach einem der Ansprüche 1 bis 3 für die Herstellung einer dermatologischen Zusammensetzung, die die Immunabwehr der Haut verstärken soll.
